# EUROPEAN PATENT SPECIFICATION

(11) **EP 3 071 154 B1**
(45) Date of publication and mention of the grant of the patent: **29.08.2018**
(21) Application number: 14796888.7
(22) Date of filing: 11.09.2014
(51) Int. Cl.: A61B 17/88, A61F 2/46

(54) **DEVICE AND METHOD FOR PERFUSING POROUS BIOMATERIALS WITH BIOLOGICAL LIQUIDS**
VORRICHTUNG UND VERFAHREN ZUR DURCHSCHWEMMUNG PORÖSER BIOMATERIALEN MIT BIOLOGISCHEN FLÜSSIGKEITEN
DISPOSITIF ET PROCÉDÉ DE PERFUSION DE BIOMATÉRIAUX POREUX AVEC DES LIQUIDES BIOLOGIQUES

(30) Priority: 19.11.2013 IT VI20130277; 19.11.2013 IT VI20130278
(43) Date of publication of application: 28.09.2016
(73) Proprietor: Bioteck S.p.A., 36057 Arcugnano (VI) (IT)
(72) Inventor: FIORINI, Mauro, 40057 Granarolo dell'Emilia (BO) (IT); RAIMONDI, Fabrizio, 37060 Castel d'Azzano (VR) (IT)
(74) Representative: Maroscia, Antonio
(86) International application number: PCT/IB2014/064433
(87) International publication number: WO 2015/075573

(56) References cited:
- WO-A1-2009/125282
- US-A1- 2007 225 665
- US-A1- 2008 214 998
- US-B1- 7 530 982
- US-B1- 8 439 929

## Description

### Field of the invention

The present invention generally finds application in the field of medical devices and particularly relates to a device for perfusing porous biomaterials with biological liquids.

The invention also relates to a method of perfusing porous biomaterials with biological liquids.

### Background Art

Devices and methods are known to be used in the medical field of therapeutic treatments for perfusing a porous biomaterial with a perfusion liquid. The perfusion liquid may be selected from the group comprising aqueous solutions, bone marrow or derivatives thereof, antibiotic-containing solutions, peripheral blood, blood components or the like.

The use of perfused biological materials is particularly important in surgical treatment of muscoloskeletal tissue alterations and/or defects, for example caused by events of traumatic origin, neoplastic or degenerative diseases, delayed unions, infant deformities, or the like.

Particularly, these devices and methods promote perfusion of biomaterials designed to be grafted into the human body as substitutes for bones, cartilages, osteocartilages, tendons, ligaments or the meniscus and other connective tissues in general.

The devices and methods for perfusing biomaterials allow air to be withdrawn from the pores of the biomaterial and a given biological liquid to be filled therein to increase the contact surface and enhance liquid-biomaterial interaction.

The perfusion process must be carried out in an isolated environment, whereby devices have been developed for perfusing a biomaterial which are adapted to maintain the biomaterial and the fluid under high sterility conditions.

WO2009125282 discloses a device and a method for perfusing biomaterials which use a perfusion chamber for containing the material to be perfused, connected to a transfer chamber containing the biological liquid.

The perfusion chamber and the transfer chamber are sealingly coupled to each other and isolated from the outside environment. The transfer chamber essentially consists of a syringe having a liquid outlet in fluid communication with the perfusion chamber.

One drawback of this solution is the difficulty of releasing the air withdrawn from the biomaterial to the outside, due to full isolation of the perfusion and transfer chambers.

When the operator uses the device, he/she has to position the perfusion and transfer chambers in the vertical direction for the air that has been withdrawn from the biomaterial to expand into the end portion of the transfer chamber that has no liquid therein.

The presence of air in the transfer chamber as perfusion cycles are repeated further decreases the efficiency of the device and increases the risk that it may be reintroduced into the biomaterial to be perfused.

In view of obviating these drawbacks, devices and methods are known for perfusing biomaterial with biological liquid, which afford the air that has been withdrawn from the biomaterial during the perfusion cycle to be released to the outside.

US2008/0214998 discloses a device and method for perfusing biomaterial with a biological liquid which, in a particular embodiment, use a single perfusion chamber to contain both the material to be treated and the biological fluid.

Particularly, the device comprises a plunger held in the chamber, which is designed to be manually actuated by the user to decrease and/or increase pressure in the chamber, to assist the release of air from the porosities of the biomaterial and the introduction of liquid therein.

This device comprises a hydrophobic gasket associated with one end of the plunger and adapted to retain the liquid in the perfusion chamber and allow escape of air through the passages formed in the plunger.

A first drawback of this arrangement is that not a high fluid sealing action is provided between the hydrophobic gasket and the end of the plunger, which causes the risk of small liquid leakages during use of the device.

A further drawback of this arrangement is that the leakage of the perfusion liquid through the plunger often has to be compensated for by predetermined amounts of liquid.

This compensation may be particularly difficult because many therapeutic treatments require perfusion of the biomaterial with very small amounts of biological fluids that have been withdrawn from living patients by particularly complex and dangerous operations.

An additional drawback of this arrangement is that the compensation for such fluid leakage requires longer total perfusion times.

Another important drawback is that the fluid and the biomaterial are exposed to significant contamination due to the low fluid sealing action between the gasket and the plunger.

### Disclosure of the invention

The object of the present invention is to overcome the above drawbacks, by providing a device and a method for perfusing porous biomaterials with biological fluids, that is highly efficient and relatively cost-effective.

A particular object of the present invention is to provide a device and a method for perfusing porous biomaterials with biological liquids that afford optimized withdrawal of air from the biomaterial and saturation of the biomaterial with biological liquid.

Another object of the present invention is to provide a device and a method for perfusing porous biomaterials with biological liquids that afford high isolation of liquid from the outside environment, to considerably reduce leakages during use.

A further object of the present invention is to provide a device and a method for perfusing porous biomaterials with biological liquids that afford perfusion of the biomaterial with small amounts of liquid.

Another object of the present invention is to provide a device and a method for perfusing porous biomaterials with biological liquids that afford reduced perfusion times.

A further object of the present invention is to provide a device and a method for perfusing porous biomaterials with biological liquids that maintain the biomaterial and the liquid under aseptic conditions.

These and other objects, as clearly explained hereinafter, are fulfilled by a device for perfusing porous biomaterials with biological liquids as defined in claim 1, which comprises a tubular body with an axial cavity defining a perfusion chamber for containing a porous biocompatible material, a connector located at a distal end of the tubular body for introducing a perfusion liquid therein and a plunger with a distal end adapted to slide in the cavity to withdraw air from the biomaterial to be perfused.

Furthermore, the device comprises vent means for releasing the air withdrawn from the biomaterial and contained in the perfusion chamber to the outside, and filter means which permit selective passage of the air contained
in the chamber toward the vent means, while preventing leakage of the liquid to the outside.

The device is characterized in that it comprises air collection means which communicate with the perfusion chamber and the vent means.

In order to control air flow from the collection means to the vent means control means are provided, which are associated with the plunger and are designed to be actuated by the user to control the air flow from the collection means to the vent means.

With this arrangement, the device affords optimized withdrawal of air from the biomaterial, while maintaining high isolation of the liquid from the outside environment and hence considerably reducing leakages thereof during use, and reducing the amount of the biological liquid required for perfusion.

In another aspect, the invention provides a method of perfusing porous biomaterials as defined in claim 11.

Advantageous embodiments of the invention are as defined in the dependent claims.

### Brief description of the drawings

Further features and advantages of the invention will be more readily apparent upon reading of the detailed description of a preferred, non-exclusive embodiment of a device and a method for perfusing a porous biomaterial with perfusion liquids or the like, which are shown as a nonlimiting example with the help of the annexed drawings, in which:

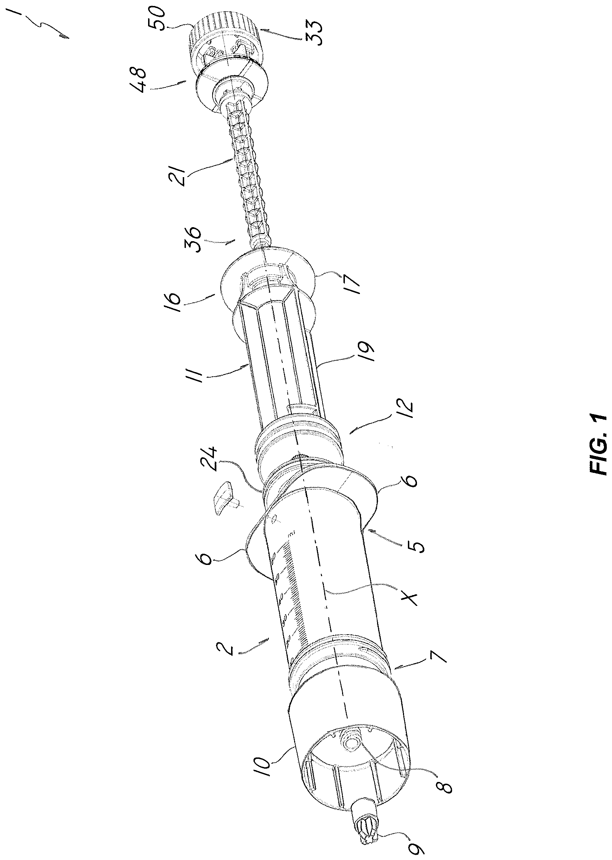

FIG. 1 is an exploded perspective view of a device of the invention;

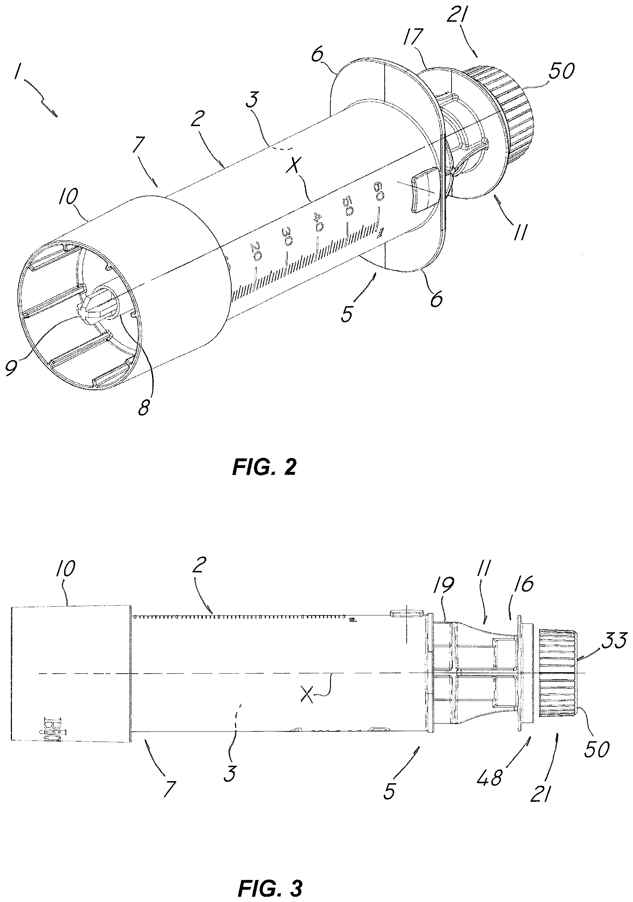

FIG. 2 is an assembled perspective view of the device of Fig. 1;
FIG. 3 is a lateral view of the device of Fig. 1;
FIG. 4 is a broken away lateral view of the device of Fig. 1 in a first operating position;
FIG. 5 is an enlarged lateral view of a first portion of Fig. 4;
FIG. 6 is an enlarged lateral view of a second portion of Fig. 4;
FIG. 7 is a broken away lateral view of the device of Fig. 1 in a second operating position;
FIG. 8 is a broken away lateral view of the device of Fig. 1 in a third operating position;
FIG. 9 is a broken away front view of the device of Fig. 1;

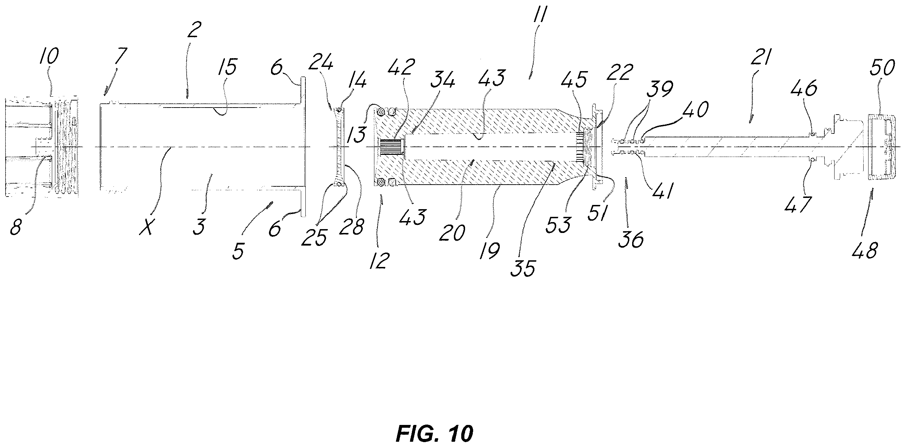

FIG. 10 is a broken away and exploded lateral view of the device of Fig. 1;
FIG. 11 is a block diagram of the perfusion method of the invention.

### Detailed description of a preferred embodiment

With reference to the above mentioned figures a device is shown, generally designated by numeral 1, for perfusing a porous biomaterial P with biological liquids L.

Particularly, the device 1 may be used for treating porous biomaterials P of a variety of types and shapes, which are designed to be grafted into the human body as substitutes for bones, cartilages, osteocartilages, tendons, ligaments or the meniscus or other connective tissues in general.

Perfusion liquids L which are typically used may be of biological origin, such as peripheral blood and derivatives thereof, bone marrow, bone marrow concentrate, adipose tissue-derived cell concentrates, physiological solutions containing antibiotics or other similar drugs.

In the embodiment of the figures, the device 1 is in syringe-like form and comprises a tubular body 2 with a longitudinal axis X and an axial cavity 3 defining a perfusion chamber 4 for holding a porous biomaterial P to be perfused.

The tubular body 2 has an open proximal end 5, having a pair of diametrically-opposed wings 6 for gripping by a user, and a distal end 7 with a connector 8 for introducing a perfusion liquid L into the perfusion chamber 4. The connector 8 may be closed off with a Luer lock cap 9 of per se known type.

Particularly, the connector 8 may be formed on a closing cover 10, which is removably mounted to the distal end 7 of the tubular body 2.

Advantageously, the removal of the closing cover 10 may provide access to the perfusion chamber 4 for introducing the porous biomaterial P therein before perfusion.

A plunger 11 is also provided, which may be equipped with longitudinal stiffening ribs for improved compressive and longitudinal tensile strength, and has a distal end 12 which is slidably movable in the cavity 3 for withdrawing the air contained in the biomaterial P to be perfused.

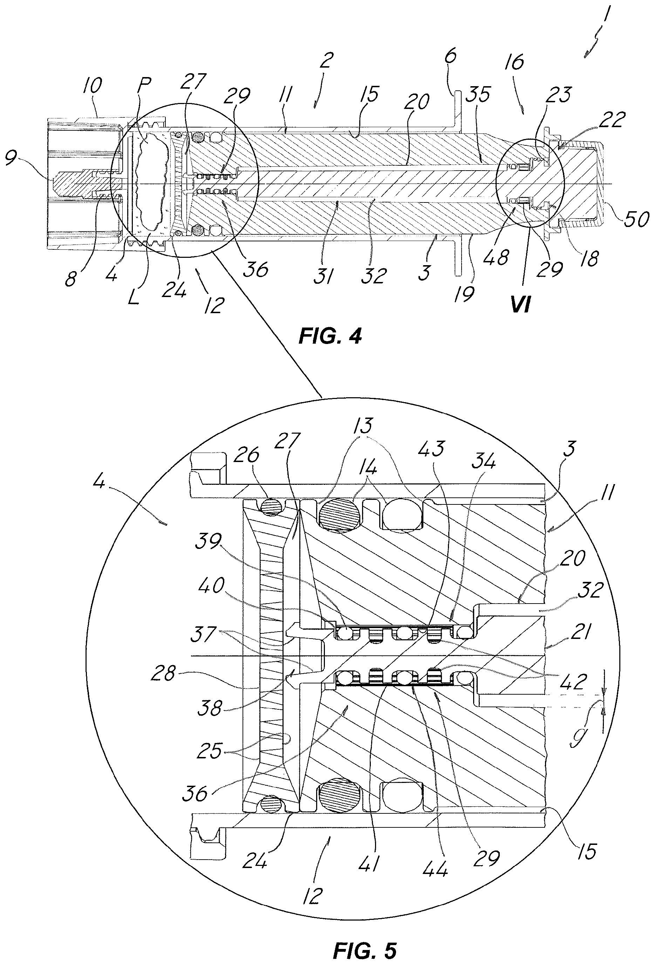

As shown in FIG. 5, an O-ring seal 14 is inserted in an annular groove 13 at the distal end of the plunger 12, for providing a sealing action on the surface 15 of the axial cavity 3 during its axial sliding motion.

In the embodiment of the figures, an annular flange 17 having a smaller size than the wings 6 is provided at the proximal end 16 of the plunger 11, for even easier gripping by a user.

The device further has vent means, generally referenced 18, for releasing the air withdrawn from the biomaterial P and collected in the perfusion chamber 4 to the outside.

The plunger 11 may comprise an elongate member 19 having a central axial through hole 20 for accommodating a stem 21 and an annular seat 22 fluidically connected to the hole 20 and located near the proximal end 16 of the plunger 11.

The fluid communication between the annular seat 22 and the axial hole 20 is ensured by the vent means 18 that shall permit air removal.

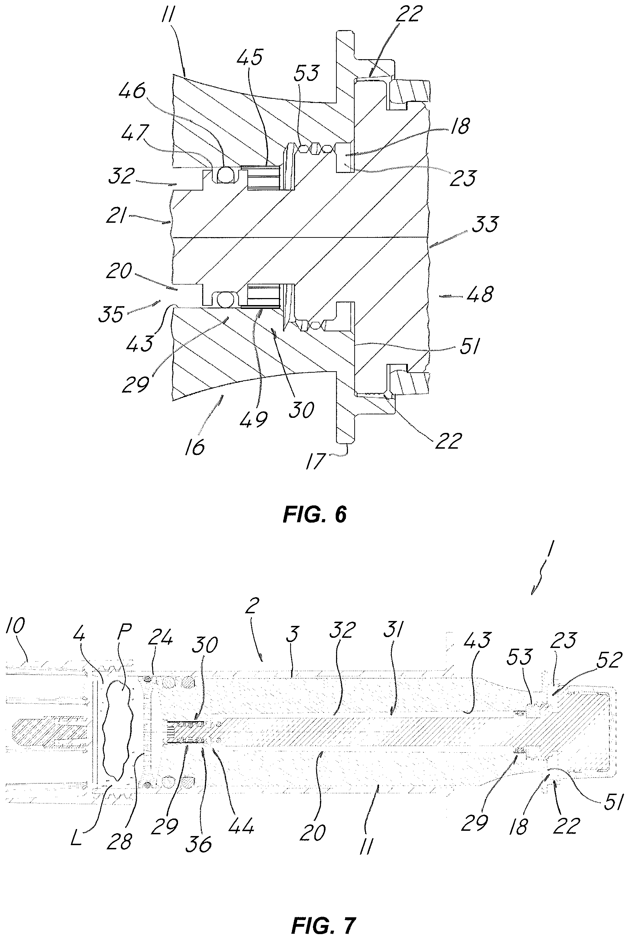

As best shown in FIG. 6, the vent means 18 may comprise an annular passageway 23, also formed at the distal end 16 of the plunger 11.

Conveniently, the perfusion chamber 4 may be defined by a substantially transverse partition 24 which is designed to be positioned in the cavity 3.

The partition 24 may substantially have a disk shape with a pair of slightly concave faces 25 facing the distal end 7 of the tubular body 2 and the plunger 11 respectively, and an annular peripheral seal 26, also of the O-ring type, for sealingly locking it in a predetermined axial position.

The distal end 12 of the plunger 11 may be slightly concave and spaced from the partition 24 to define a suction chamber 27 having a predetermined volume V₁ for containing the liquid L with the air that has been withdrawn from the biomaterial P.

Conveniently, the axial position of the partition 24 may be appropriately adjusted to change the volume V₂ of the perfusion chamber 4.

Thus, the volume V₂ of the chamber 4 may be adapted to the actual maximum size of the porous biomaterial P, thereby allowing minimization of the amount of liquid L required for perfusion thereof.

Preferably, the partition 24 may comprise at least one calibrated orifice 28 for promoting fluid communication of the perfusion chamber 4 with the suction chamber 27.

Filter means, generally referenced 29, are also provided, for permitting selective passage of the air contained in the perfusion chamber 4 toward the vent means 18, while preventing discharge or leakage of the perfusion liquid L to the outside.

Particularly, the filter means 29 may comprise at least one filter element 30 located downstream from the orifice 28.

The selected passage of air through the filter means 29 is facilitated by the longitudinal motion of the plunger 11 in the axial cavity 3.

When the plunger 11 is stopped, it may move away from the partition 24 thereby creating a negative pressure in the space vacated by the plunger 11.

Thus, a part of the liquid L contained in the perfusion chamber 4 will be sucked back into the suction chamber 27 through the orifice 28.

The later movement of the plunger 11 toward the partition 24 will pressurize the liquid L, thereby causing forced separation of the air removed from the pores of the biomaterial P through the filter means 29 that contact the liquid L.

The provision of the seal 14 along the walls of the plunger 11 will prevent liquid L from flowing back up along the walls thereof and will cause the flow of liquid L to be only conveyed toward the filter means 29.

A peculiar feature of the invention is that it comprises air collection means 31 which communicate with the perfusion chamber 4 and the vent means 18.

Particularly, the collection means 31 may consist of a substantially annular air space 32 in communication with the outside environment.

The air space 32 may be defined by a predetermined radial clearance g between the through hole 20 and the stem 21 upon insertion of the latter.

According to a further peculiar aspect of the invention, control means 33 are associated with the plunger 11 and are designed to be actuated by a user to control the air flow from the collection means 31 to the vent means 18.

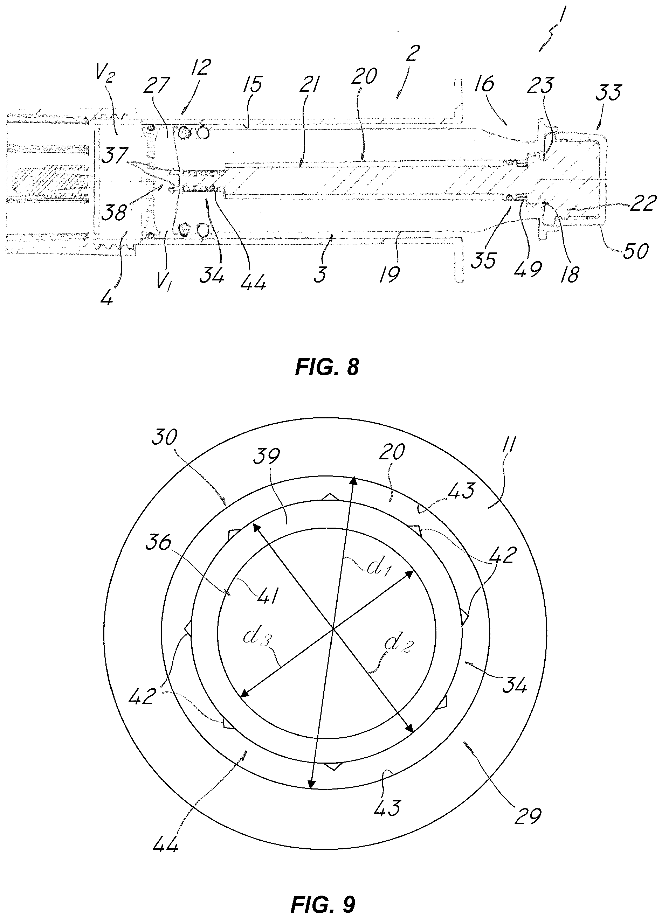

Conveniently, as best shown in FIG. 9, the through hole 20 may have a substantially cylindrical shape, with a substantially uniform first inside diameter d₁, and may have a distal end portion 34, with a second inside diameter d₂, which is smaller than the first, and a proximal end portion 35.

The distal portion 34 may be adapted to accommodate a corresponding distal end portion 36 of the stem 21 having a third outside diameter d₃ which is slightly smaller than the second inside diameter d₂.

The distal end portion 36 accommodated in the distal portion 34 of the hole 20 may be provided with tapering lock projections 37 and a diametrical slit 38, for the projections 37 to be able to bend inwards and snap into the distal end portion 34 of the hole 20 of the plunger 11.

Advantageously, as best shown in FIG. 5, the filter means 29 may comprise a first annular seal 39 accommodated in a first annular seat 40 formed on the cylindrical wall 41 of the distal end portion 36 of the stem 21.

Furthermore, the filter means 29 may comprise a plurality of first longitudinal micro-channels 42 formed in angularly offset positions on the inner wall 43 of the distal end portion 34 of the through hole 20 of the plunger 11.

Thus, the first seal 39 may interact with the first micro-channels 42 to form a first labyrinth filter 44.

The radially offset arrangement of the micro-channels 42 may be better understood with the help of FIG. 9, although such micro-channels are not drawn to scale with respect to the dimensions of the other parts of the device.

Advantageously, the filter means 29 may comprise a plurality of second longitudinal micro-channels 45 formed in angularly offset positions on the inner wall 43 of the proximal portion 35 of the through hole 20, and at least one second seal 46 accommodated in a second annular seat 47 of the proximal end 48 of the stem 21.

Particularly, in a similar manner as the first seal 39, the second seal 46 may interact with the second micro-channels 45 to form a second labyrinth seal 49 which is longitudinally offset from the first filter.

Conveniently, both the first 42 and second 45 micro-channels may have micrometric dimensions, for example equal to or smaller than 100 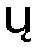 m, and are adapted to facilitate selective passage of air bubbles.

Preferably, the control means 33 may comprise an operating ring 50 located at the proximal end 48 of the stern 21 and adapted to be sealingly inserted in the annular seat 22 of the plunger 11.

Particularly, the stem 21 is movable within the through hole 20 of the plunger 11 between a forward position, as shown in FIG. 4, in which the ring 50 contacts the bottom wall 51 of the annular seat 22 and blocks the through hole 20, and a backward position, as shown in FIG. 7, in which the ring 50 is separate from the seat 22 and opens the passageway 23 thereby defining a vent 52.

Conveniently, the stem 21 may have a locking member 53 near its proximal end 48, which is adapted to removably join the plunger 11 to the stem 21 when the latter is in the forward position.

The locking member 53 may allow the plunger 11 to reciprocate to create a negative pressure in the suction chamber 27 and convey air into the air space 32.

Furthermore, the locking member 53 may be releasable to move the stem 21 to the backward position and allow the air collected in the air space 32 to be released through the vent port 52.

Conveniently, the stem 21 may be moved between the forward position and the backward position a number of times to remove the air that has been previously withdrawn from the biomaterial P, while preventing it from being reintroduced into the pores thereof during the next perfusion cycle.

This arrangement increases the amount of liquid L that fills the pores of the biomaterial P thereby improving the overall efficiency of the perfusion process.

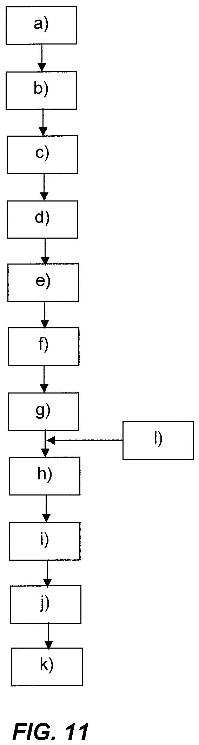

In a further aspect the invention relates to a method of perfusing biomaterials with biological liquids, as best shown in the block diagram of FIG. 11, which is adapted to be implemented by the device 1 for perusing a porous biomaterial P with biological liquids L.

The method basically comprises a step a) of providing the tubular body 2 with the inner cavity 3 defining the perfusion chamber 4.

Preferably, the closing cover 10 may be provided at the distal end 7 of the tubular body 2.

Once the cover 10 has been removed for access to the chamber 4 of the tubular body 2, a step b) will follow, in which a porous biomaterial P is placed in the chamber 4.

Possibly, the volume of the perfusion chamber 4 may be changed to fit the volume of the porous biomaterial P to be perfused by positioning the transverse partition 24, which is longitudinally translatable within the cavity 3.

This will optimize the volume of the chamber 4, and maximize the volume and amount of liquid L required for filling it.

Once the porous biomaterial P has been introduced into the perfusion chamber 4, the cover 10 may be mounted back to the distal end 7 of the tubular body 2 and the step c) of filling the chamber 4 with the perfusion liquid L may be carried out.

For this purpose, the connector 8 may be provided on the cover 10, for fluid communication with an external reservoir containing the biological liquid I, not shown, through a tube.

The chamber 4 containing the biomaterial P to be perfused may be filled by gravity. Alternatively, the perfusion chamber 4 may be filled using appropriate pumping means.

The step c) of filling the perfusion chamber 4 with the biological liquid L may be carried out under totally sterile conditions, for stable isolation of the liquid L from the outside environment.

Once the chamber 4 has been filled and the external tube has been removed, the connector 8 may be closed again using a Luer lock cap 9 of the per se known type, to prevent leakage of liquid L from the chamber 4 to the outside.

Then a step d) may be provided, in which the axially movable plunger 11 with the axial through hole 20 is introduced into the cavity 3 of the tubular body 2.

Conveniently, the plunger 11 is selected with an outside diameter that is slightly smaller than the inside diameter of the tubular body 2 and has O-rings 14 toward its distal end 12, such that it may sealingly slide in the inner cavity.

The method also includes a step e) in which the rigid stem 21 is accommodated in the axial hole 20.

The stem 21 is positioned in the axial hole 20 with a predetermined clearance, to thereby define the air collection space 32, in fluid communication toward its distal end 12 with the perfusion chamber 4, and toward the proximal end 16 of the plunger 11 with the outside.

Furthermore, the stem 21 in the axial hole 20 may have control means 33 for controlling fluid communication with the outside.

The control means 33 promote the release of withdrawn air to the outside during the perfusion process.

The method also comprises a step f) in which the stem 21 is positioned, relative to the plunger 11, in a forward locking position to prevent the passage of air from the air space 32 to the outside and facilitate mutual sliding thereof.

The stem 21 may be removably locked relative to the plunger 11 by separable locking members 53 which are located near the proximal end 16 of the plunger 11 and are designed to be accessed by a user.

Preferably, the locking step f) may be carried out by helical engagement of threads formed on the proximal end 36 of the inner wall 43 of the axial hole 20 of the plunger 11 with mating threads formed on the cylindrical wall of the proximal end 48 of the stem 21.

After the locking step f), the method comprises a step g) in which the stem-plunger assembly in locked relationship are reciprocated to repeatedly create suctions and compressions in the perfusion chamber 4.

The reciprocating motion of the assembly will pressurize the biological liquid L in the perfusion chamber 4 and cause it to effectively penetrate the pores of the biomaterial P.

Furthermore, once air has been withdrawn from the porous biomaterial P, the repeated reciprocating motion of the stem-plunger assembly will facilitate separation of air bubbles from the liquid L and conveyance thereof into the air collection space 32.

Advantageously, this arrangement will collect air in the air space 32 and prevent any communication of the inner portions of the tubular body 2 with the outside.

Preferably, the reciprocating motion of the assembly may be repeated as many times as is required for complete removal of air from the pores of the biomaterial P and the biological liquid L and for consequent saturation of the biomaterial P with the liquid.

Furthermore, as suction and compression steps are alternately repeated, the user will firmly tap his/her hand palm on the tubular body 2 to facilitate separation of the air bubbles that are still in the pores of the biomaterial P due to cavitation.

At the end of the perfusion process, the method includes a step h) in which the stem 21 is placed in a backward unlocking position relative to the plunger 11 to allow communication of the air space 32 with the outside and release air therein.

The stem 21 may be moved by providing and longitudinally displacing an operating ring 50 connected to the proximal end 48 of the stern 21. Advantageously, the ring 50 of the stem 21 may define the control means 33 for selectively promoting communication of the air collection space 32 with the outside.

Conveniently, communication between the air collection space 32 and the outside may take place thanks to the passageway 23 provided at the proximal end 16 of the plunger 11, where a seat 22 for the operating ring 50 may be also conveniently provided.

Advantageously, the passageway 23 may be closed by moving the stem 21 forward and entirely introducing the ring 50 into its seat 22.

Conversely, the passageway 23 may be opened by retracting the stem 21 and moving the ring 50 away from the seat 22 for the air contained in the air space to be released to the outside.

Controlled retraction of the stem 21 may be repeated by the user until the air contained in the air collection space 32 is entirely exhausted to the outside environment.

The method also includes a step i) in which the steps g) and h) are repeated as many times as is required for approximately complete removal of air from the pores of the biomaterial P and expulsion thereof.

After the repetition step i), there will be a step j) in which the liquid L is drained from the perfusion chamber 4 and a step k) in which the perfusion chamber 4 is opened and the completely treated porous biomaterial P is removed therefrom.

Preferably, as air enters and exits the air space 32, the method includes respective filtering steps l) for separating the air that has been withdrawn from the porous biomaterial P from the biological liquid L.

The filtering steps l) may be carried out using the longitudinal micro-channels 42, 45 that are formed at the end zones 34, 35 of the axial hole 20 and one or more annular seals 39, 46 mounted to the corresponding areas 36, 48 of the stem 21.

Particularly, these filtering steps l) may be carried out by interaction of the micro-channels 42, 45 with the annular seals 39, 46 such that micro-metric openings are created, to prevent leakage of liquids and allow selective passage of air.

The annular seals 39, 46 are adapted to directly interact with the inner wall 43 of the axial hole 20 to ensure a sealing action against leakage of biological liquid L toward the air space 32 and simultaneous formation of micro-metric openings for the air that has been withdrawn from the porous material P.

The device and the method for perfusing a porous biomaterial of the invention are susceptible of many changes and variants within the inventive principle disclosed in the annexed claims.

All the details and procedures thereof may be replaced by other technically equivalent parts and methods, and the materials may vary depending on different needs, without departure from the scope of the invention as defined by the appended claims.

While the device and method of the invention have been described with particular reference to the annexed figures, the numerals referred to in the disclosure and claims are only used for the sake of a better intelligibility of the invention and shall not be intended to limit the claimed scope in any manner.

### Industrial Applicability

The present invention finds industrial application in the field of medical devices and particularly for the provision of a device and method for perfusing porous biomaterials with biological liquids.

## Claims

1. A device for perfusing porous biomaterials with biological liquids, comprising:
- a tubular body (2) with an axial cavity (3) defining a perfusion chamber (4) for containing a porous biocompatible material (P);
- a connector (8) located at a distal end (7) of said tubular body (2) for introducing a perfusion liquid (L) therein;
- a plunger (11) having a distal end (12) slidably movable in said cavity (3) to withdraw air from the biomaterial (P) to be perfused;
- vent means (18) for releasing the air withdrawn from the biomaterial (P) and contained in said chamber (4) to the outside;
- filter means (29) for permitting selective passage of the air contained in said chamber (4) toward said vent means (18), while preventing leakage of the liquid (L) to the outside;
**characterized in that** it comprises air collection means (31), which communicate with said chamber (4) and said vent means (18), control means (33) being associated with said plunger (11) and designed to be actuated by the user to control the air flow from said collection means (31) to said vent means (18).

2. A device as claimed in claim 1, wherein said plunger (11) comprises an elongate element (19) having an axial through hole (20) and adapted to accommodate a stem (21), and a seat (22) located near the proximal end (16) of said elongate element (19), said seat (22) and said through hole (20) being fluidically connected through said vent means (18) comprising an annular passageway (23).

3. A device as claimed in claim 2, wherein said stern (21) has a predetermined radial clearance (g) relative to said axial through hole (20) to define a substantially annular air space (32) therewith, said collection means (31) consisting of said air space (32).

4. A device as claimed in claim 2, wherein said control means (33) comprise an operating ring (50) located at the proximal end (48) of said stem (21) and adapted to be sealingly introduced into said annular seat (22) of said plunger (11).

5. A device as claimed in claim 2, wherein said stem (21) is movable within said through hole (20) of said plunger (11) between a forward position, in which said ring (50) contacts the bottom wall (51) of said seat (22) and blocks said through hole (20), and a backward position, in which said ring (50) is separate from said seat (22) and opens said passageway (23) thereby defining a vent (52).

6. A device as claimed in claim 2, wherein said axial hole (20) has a substantially cylindrical shape, with a substantially uniform first inside diameter (d₁) and has a distal end portion (34), with a second inside diameter (d₂), which is smaller than the first, and a proximal end portion (35), said distal portion (34) being adapted to accommodate a corresponding distal end portion (36) of said stem (21) having a third outside diameter (d₃) which is slightly smaller than said second inside diameter (d₂).

7. A device as claimed in claim 2, wherein said chamber (4) is defined by a substantially transverse partition (24) which is designed to be adjustably positioned in said cavity (3) to adapt the volume (V₂) of said chamber (4) to the maximum size of the biomaterial (P) and minimize the amount of perfusion liquid (L), the distal end (12) of said plunger (11) being slightly concave and spaced from said partition (24) to define a suction chamber (27) for sucking in liquid containing the air withdrawn from the biomaterial (P) to be perfused, said partition (24) having at least one calibrated orifice (28) which is adapted to allow communication between said perfusion chamber (4) and said suction chamber (27).

8. A device as claimed in claim 2, wherein said filter means (29) comprise a plurality of first longitudinal micro-channels (42) formed in angularly offset positions on the inner wall (43) of said distal end portion (34) of said hole (20), and at least one first annular seal (39) accommodated in a first annular seat (40) of the distal end portion (36) of said stem (21) and adapted to interact with said first micro-channels (42) to form a first labyrinth filter (44).

9. A device as claimed in claim 8, wherein said filter means (29) comprise a plurality of second longitudinal micro-channels (45) formed in angularly offset positions on the inner wall (43) of said proximal portion (35) of said hole (20), and at least one second seal (46) accommodated in a second annular seat (47) of the proximal portion (48) of said stem (21) and adapted to interact with said second micro-channels (45) to form a second labyrinth filter (49), which is longitudinally offset from the first.

10. A device as claimed in claim 2, wherein said stern (21) has a locking member (53) near said proximal end (48) for removably joining said plunger (11) to said stem (21), with the latter being in the forward position, and for allowing said plunger (11) to reciprocate to create a negative pressure in said suction chamber (27) and convey air into said air space (32), said locking member (53) being releasable to move said stern (21) to said backward position and allow the air collected in said air space (32) to be released through a vent (52)

11. A method of perfusing porous biomaterials (P) with biological liquids (L), comprising the steps of:
a) providing a tubular body (2) with an inner cavity (3) defining a perfusion chamber (4);
b) positioning a porous biomaterial (P) in said chamber (4);
c) filling said chamber (4) with a perfusion liquid (L);
d) introducing an axially movable plunger (11), having an axial through hole (20), into said cavity (3);
e) accommodating a stem (21) in said hole (20), with a predetermined clearance to define an air collection space (32) communicating with said chamber (4) and with the outside and having control means (33) for controlling communication with the outside;
f) positioning said stem (21), relative to said plunger (11), in a forward locking position to prevent the passage of air from the air space (32) to the outside;
g) reciprocating the stem-plunger assembly in locked relationship to create repeated suctions and compressions in said chamber (4) and promote separation of air bubbles from the liquid and conveyance thereof toward said air collection space (32);
h) positioning said stem (21) relative to said plunger (11) in a backward unlocking position to permit communication of said air space (32) with the outside and release the air collected therein;
i) repeating the steps g) and h) as many times as is required for approximately complete removal of air from the pores of the porous biomaterial (P) and filling of the latter with the perfusion liquid (L);
j) draining the liquid (L) from the perfusion chamber (4);
k) opening the perfusion chamber (4) and removing the completely treated porous material (P).

12. A method as claimed in claim 11, wherein a plurality of longitudinal micro-channels (42, 45) are provided at the entry and exit of said air space (32), and are formed at the end areas (34, 35) of said axial hole (20), and one or more annular seals (39, 49) are provided on the corresponding areas (36, 48) of said stem (21), to form micro-metric openings, filtering steps I) being provided in which the perfusion liquid (L) is filtered by said micro-metric openings to prevent leakage of liquids and allow selective passage of air.

13. A method as claimed in claim 11, wherein said stem (21) is locked relative to said plunger (11) by a separable locking member (53) which is located near the proximal end (16) of the plunger (11) and is designed to be accessed by a user.

14. A method as claimed in claim 11, wherein the backward and forward motion of said stem (21) in the axial hole (20) are obtained by providing an operating ring (50) connected to the proximal end (48) of said stem (21) and longitudinally moving it by the user.

15. A method as claimed in claim 14, wherein an annular passageway (23) is provided between said air space (32) and the outside at said proximal end (16) of said plunger (11) and a seat (22) is provided for said ring (50) at the exit of said passageway (23),
said passageway (23) being closed by moving said stem (21) forward and entirely introducing said ring (50) into said seat (22), the air collected in said air space (32) being released to the outside by moving said stem (21) backward and moving said ring (50) away from said seat (22) thereby opening said passageway (23).

## Patentansprüche

1. Ein Gerät zur Perfusion poröser Biomaterialien mit biologischen Flüssigkeiten, einschließlich:
- eines rohrförmigen Körpers (2) mit einem axial verlaufenden Hohlraum (3), der eine Perfusionskammer (4) bildet, zur Aufnahme eines porösen biokompatiblen Materials (P);
- einem Anschluss (8) an einem distalen Ende (7) des genannten rohrförmigen Körpers (2) zum Einleiten einer Perfusionsflüssigkeit (L);
- einem Kolben (11) mit einem distalen Ende (12), der durch Verschieben in dem genannten Hohlraum (3) bewegt werden kann, um die Luft aus dem zu durchströmenden Biomaterial (P) zu ziehen;
- Lüftungseinrichtungen (18) zur Abgabe der aus dem Biomaterial (P) herausgezogenen und in der genannten Kammer (4) enthaltenen Luft nach außen;
- Filtervorrichtungen (29) zur Ermöglichung eines selektiven Durchflusses der in der genannten Kammer (4) enthaltenen Luft zu den Lüftungseinrichtungen (18), während ein Auslaufen der Flüssigkeit (L) nach außen verhindert wird;
**dadurch gekennzeichnet, dass** es Vorrichtungen zum Sammeln der Luft (31) umfasst, die mit der genannten Kammer (4) und den genannten Lüftungseinrichtungen (18) verbunden sind, wobei die mit dem genannten Kolben (11) verbundenen und zur Betätigung durch den Benutzer ausgelegten Steuermittel (33) zur Steuerung des Luftstroms von den genannten Vorrichtungen zum Sammeln der Luft (31) zu den genannten Lüftungseinrichtungen (18).

2. Gerät gemäß Anspruch 1, **dadurch gekennzeichnet, dass** der genannte Kolben (11) ein längliches Element (19) mit einem axialen Durchgangsloch (20) aufweist, das zur Aufnahme eines Schaftes (21) und eines Sitzes (22) in der Nähe des proximalen Endes (16) des genannten länglichen Elementes (19) geeignet ist, wobei der genannte Sitz (22) und das genannte Durchgangsloch (20) strömungstechnisch über die genannten Lüftungseinrichtungen (18) einschließlich eines ringförmigen Durchgangs (23) verbunden sind.

3. Gerät gemäß Anspruch 2, **dadurch gekennzeichnet, dass** der genannte Schaft (21) einen vorab festgelegten radialen Freiraum (g) bezüglich des genannten axialen Durchgangslochs (20) aufweist, um damit einen im Wesentlichen ringförmigen Luftraum (32) zu bilden, d.h. die genannten Vorrichtungen zum Sammeln der Luft (31), die aus dem genannten Luftraum (32) bestehen.

4. Gerät gemäß Anspruch 2, **dadurch gekennzeichnet, dass** die genannten Steuermittel (33) einen Einsetzring (50) am proximalen Ende (48) des genannten Schafts (21) umfassen, der geeignet ist, um als Abdichtung in den genannten ringförmigen Sitz (22) des genannten Kolbens (11) eingefügt zu werden.

5. Gerät gemäß Anspruch 2, **dadurch gekennzeichnet, dass** der genannte Schaft (21) mit dem genannten Durchgangsloch (20) des genannten Kolbens (11) zwischen einer vorderen Position, in der der genannte Ring (50) die untere Wandung (51) des genannten Sitzes (22) berührt und das genannte Durchgangsloch (20) verschließt, und einer hinteren Position bewegt werden kann, in der der genannte Ring (50) vom genannten Sitz (22) gelöst ist und den genannten Durchgang (23) öffnet, wodurch ein Abzug (52) entsteht.

6. Gerät gemäß Anspruch 2, **dadurch gekennzeichnet, dass** das genannte axiale Durchgangsloch (20) eine im Wesentlichen zylinderförmige Form mit einem im Wesentlichen gleichmäßigen ersten Innendurchmesser (d₁), einem distalen Endabschnitt (34) mit einem zweiten Innendurchmesser (d₂), der kleiner als der erste ist, und einen proximalen Endabschnitt (35) besitzt, wobei der genannte distale Abschnitt (34) angepasst wurde, um einen entsprechenden distalen Endabschnitt (36) des genannten Schaftes (21) mit einem dritten Außendurchmesser (d₃) aufzunehmen, der geringfügig kleiner als der zweite Innendurchmesser (d₂) ist.

7. Gerät gemäß Anspruch 2, **dadurch gekennzeichnet, dass** die genannte Kammer (4) durch eine im Wesentlichen schräge Aufteilung (24) gekennzeichnet ist, die im genannten Hohlraum (3) verstellbar gestaltet ist, um das Volumen (V₂) der genannten Kammer (4) der maximalen Größe des Biomaterials (P) anzupassen sowie die Menge der Perfusionsflüssigkeit (L) zu minimieren, dass das distale Ende (12) des genannten Kolbens (11) leicht konkav ist und von der genannten Aufteilung (24) entfernt ist, um eine Ansaugkammer (27) zum Ansaugen der die aus dem Biomaterial (P) abgesaugte Luft enthaltenden Perfusionsflüssigkeit zu bilden, wobei die genannte Aufteilung (24) mindestens eine kalibrierte Öffnung (28) besitzt, die angepasst wurde, um eine Verbindung zwischen der genannten Perfusionskammer (4) und der genannten Ansaugkammer (27) zu ermöglichen.

8. Gerät gemäß Anspruch 2, **dadurch gekennzeichnet, dass** die genannten Filtervorrichtungen (29) eine Vielzahl erster Mikro-Kanäle (42) in Längsrichtung umfassen, die in winkelförmiger versetzten Position auf der Innenwand (43) des genannten distalen Endabschnitts (34) des genannten Durchgangslochs (20) gebildet werden, und mindestens einen ersten Dichtungsring (39) umfassen, der in einen ersten ringförmigen Sitz (40) des distalen Endabschnitts (36) des genannten Schaftes (21) eingelegt und angepasst wurde, um mit den ersten Mikro-Kanälen (42) einen ersten Labyrinth-Filter (44) zu bilden.

9. Gerät gemäß Anspruch 8, **dadurch gekennzeichnet, dass** die genannten Filtervorrichtungen (29) eine Vielzahl zweiter Mikro-Kanäle (45) in Längsrichtung umfassen, die in winkelförmiger Position auf der Innenwand (43) des genannten proximalen Endabschnitts (35) des genannten Durchgangslochs (20) gebildet werden, und mindestens eine zweite Dichtung (46) umfassen, die in einen zweiten ringförmigen Sitz (47) des proximalen Endabschnitts (48) des genannten Schaftes (21) eingelegt und angepasst wurde, um mit den zweiten Mikro-Kanälen (45) einen ersten Labyrinth-Filter (49) zu bilden, der in Längsrichtung zum ersten versetzt ist.

10. Gerät gemäß Anspruch 2, **dadurch gekennzeichnet, dass** der genannte Schaft (21) ein Blockierelement (53) am genannten proximalen Ende (48) aufweist, um den genannten Kolben (11) mit dem genannten Schaft (21) abnehmbar zu verbinden, wobei sich der Letztere in der vorderen Position befindet, und um es zu ermöglichen, dass der genannte Kolben (11) beim Zurückziehen einen Unterdruck in der genannten Ansaugkammer (27) aufbaut und die Luft in den genannten Luftraum (32) fließt, wobei das genannte Blockierelement (53) lösbar ist, um den genannten Schaft (21) in die genannte hintere Position zu bewegen, damit die im genannten Luftraum (32) angesammelte Luft über eine Abzugsvorrichtung (52) abgelassen werden kann.

11. Verfahren zur Perfusion poröser Biomaterialien (P) mit biologischen Flüssigkeiten (L), das die folgenden Schritte umfasst:
a) Bereitstellen eines rohrförmigen Körpers (2) mit einem axial verlaufenden Hohlraum (3), der eine Perfusionskammer (4) bildet;
b) Positionieren eines porösen Biomaterials (P) in der genannten Kammer (4);
c) Füllen der genannten Kammer (4) mit einer Perfusionsflüssigkeit (L);
d) Einführen eines axial beweglichen Kolbens (11) mit einem axialen Durchgangsloch (20) in den genannten Hohlraum (3);
e) Einführen eines Schafts (21) in dem genannten Durchgangsloch (20) mit einem vorab festgelegten Abstand, um einen Luftsammelraum (32) zu bilden, der mit der genannten Kammer (4) und mit dem Äußeren verbunden ist, sowie über Steuermittel (33) zur Steuerung der Verbindung mit dem Äußeren verfügt;
f) Positionieren des genannten Schafts (21) bezüglich des genannten Kolbens (11), in einer vorderen Blockierposition, um das Austreten der Luft aus dem Luftraum (32) ins Äußere zu verhindern;
g) Zurückziehen der Baugruppe Schaft-Kolben in verbundener Form, um wiederholte Saug- und Komprimiervorgänge in der genannten Kammer (4) hervorzurufen und die Trennung der Luftblasen von der Flüssigkeit sowie deren Weiterleitung in den genannten Luftsammelraum (32) zu fördern;
h) Positionieren des genannten Schafts (21) bezüglich des genannten Kolbens (11), in einer hinteren nicht blockierten Position, um die Verbindung des genannten Luftraums (32) mit dem Äußeren zu ermöglichen sowie die darin gesammelte Luft abzulassen;
i) Wiederholen der Schritte g) und h) so oft, wie für die annähernd vollständige Entfernung der Luft aus den Poren des porösen Biomaterials (P) erforderlich ist, und Füllen der Letzteren mit der Perfusionsflüssigkeit (L);
j) Ablassen der Flüssigkeit (L) aus der Perfusionskammer (4);
k) Öffnen der Perfusionskammer (4) und vollständiges Entfernen des behandelten porösen Materials (P).

12. Verfahren gemäß Anspruch 11, **dadurch gekennzeichnet dass** eine Vielzahl von Mikro-Kanälen (42, 45) in Längsrichtung am Eingang und Ausgang des genannten Luftraum (32) vorhanden sind und an den Endbereichen (34, 35) des genannten axialen Durchgangslochs (20) gebildet sind, und ein oder mehrere Dichtungsringe (39, 49) sind in den entsprechenden Bereichen (36, 48) des genannten Schafts (21) vorhanden, um mikro-metrische Öffnungen zu bilden, Filterstufen l) sind vorhanden, in denen die Perfusionsflüssigkeit (L) durch die genannten mikro-metrischen Öffnungen gefiltert wird, um ein Auslaufen von Flüssigkeiten zu verhindern und einen selektiven Durchfluss der Luft zu ermöglichen.

13. Verfahren gemäß Anspruch 11, **dadurch gekennzeichnet dass** der genannte Schaft (21) mit dem genannten Kolben (11) durch eine lösbare Blockiervorrichtung (53) verbunden ist, die sich in der Nähe des proximalen Endes (16) des Kolbens befindet (11) und von einem Benutzer erreichbar ist.

14. Verfahren gemäß Anspruch 11, **dadurch gekennzeichnet dass** die Rückwärts- und Vorwärtsbewegung des genannten Schafts (21) im axialen Durchgangsloch (20) durch das Vorhandensein eines Einsetzringes (50), der mit dem proximalen Ende (48) des genannten Schafts (21) verbunden ist und vom Benutzer in Längsrichtung bewegt wird.

15. Verfahren gemäß Anspruch 14, **dadurch gekennzeichnet dass** ein ringförmiger Durchgang (23) zwischen dem genannten Luftraum (32) und dem Äußeren am genannten proximalen Ende (16) des genannten Kolbens (11) und ein Sitz (22) für den genannten Ring (50) am Ausgang des genannten Durchgangs (23) vorhanden ist, wobei der genannte Durchgang (23) durch das Bewegen des genannten Schafts (21) vorwärts und das vollständige Einfügen des genannten Rings (50) in den genannten Sitz (22) verschlossen wird, wodurch die in dem genannten Luftraum (32) gesammelte Luft durch Bewegen des genannten Schafts (21) rückwärts und Entfernen des genannten Rings (50) aus dem genannten Sitz (22) beim damit erfolgenden Öffnen des genannten Durchgangs (23) ins Äußere abgelassen wird.

## Revendications

1. Un dispositif pour perfuser des biomatériaux poreux avec des liquides biologiques, comprenant :
- un corps tubulaire (2) avec une cavité axiale (3) définissant une chambre de perfusion (4) pour contenir un matériau biocompatible poreux (P);
- un connecteur (8) situé à une extrémité distale (7) dudit corps tubulaire (2) pour introduire un liquide de perfusion (L) dans celui-ci ;
- un piston (11) ayant une extrémité distale (12) déplaçable de façon coulissante dans ladite cavité (3) pour aspirer de l'air depuis le biomatériau (P) à perfuser ;
- des moyens d'aération (18) pour libérer à l'extérieur l'air aspiré depuis le biomatériau (P) et contenu dans ladite chambre (4) ;
- des moyens de filtration (29) pour permettre le passage sélectif de l'air contenu dans ladite chambre (4) vers lesdits moyens d'aération (18), tout en évitant la fuite du liquide (L) à l'extérieur ;
**caractérisé en ce qu'**il comprend des moyens de collecte d'air (31) communiquant avec ladite chambre (4) et lesdits moyens d'aération (18), des moyens de commande (33) étant associés audit piston (11) et conçus pour être actionnés par l'utilisateur pour commander le débit d'air depuis lesdits moyens de collecte (31) vers lesdits moyens d'aération (18).

2. Un dispositif selon la revendication 1, dans lequel ledit piston (11) comprend un élément allongé (19), comportant un trou débouchant axial (20) et apte à recevoir une tige (21), et un logement (22) situé près de l'extrémité proximale (16) dudit élément allongé (19), ledit logement (22) et ledit trou débouchant (20) étant en communication fluidique entre eux par lesdits moyens d'aération (18) comprenant a passage annulaire (23).

3. Un dispositif selon la revendication 2, dans lequel ladite tige (21) a un jeu radial prédéterminé (g) par rapport audit trou débouchant axial (20) pour définir un espace d'air sensiblement annulaire (32) avec celui-ci, lesdits moyens de collecte (31) étant constitués dudit espace d'air (32).

4. Un dispositif selon la revendication 2, dans lequel lesdits moyens de commande (33) comprennent une bague d'actionnement (50) située à l'extrémité proximale (48) de ladite tige (21) et apte à être introduite de façon étanche dans ledit logement annulaire (22) dudit piston (11).

5. Un dispositif selon la revendication 2, dans lequel ladite tige (21) est mobile à l'intérieur dudit trou débouchant (20) dudit piston (11) entre une position avant, dans laquelle ladite bague (50) est en contact avec la paroi de fond (51) dudit logement (22) et bouche ledit trou débouchant (20), et une position arrière, dans laquelle ladite bague (50) est séparée dudit logement (22) et ouvre ledit passage (23) de façon à définir une ouverture d'aération (52).

6. Un dispositif selon la revendication 2, dans lequel ledit trou axial (20) présente une forme sensiblement cylindrique, avec un premier diamètre interne sensiblement uniforme (d₁) et comporte une partie d'extrémité distale (34), avec un deuxième diamètre interne (d₂) inférieur au premier, et une partie extrémité proximale (35), ladite partie distale (34) étant apte à loger une partie d'extrémité distale correspondante (36) de ladite tige (21) présentant un troisième diamètre externe (d₃), qui est légèrement inférieur audit deuxième diamètre interne (d₂).

7. Un dispositif selon la revendication 2, dans lequel ladite chambre (4) est définie par une cloison sensiblement transversale (24), destinée à être positionnée de façon réglable dans ladite cavité (3) pour adapter le volume (V₂) de ladite chambre (4) aux dimensions maximales du biomatériau (P) et à minimiser la quantité de liquide de perfusion (L), l'extrémité distale (12) dudit piston (11) étant légèrement concave et espacée de ladite cloison (24) pour définir une chambre d'aspiration (27) pour aspirer le liquide contenant l'air prélevé du biomatériau (P) à perfuser, ladite cloison (24) comportant au moins un orifice calibré (28), apte à permettre la communication entre ladite chambre de perfusion (4) et ladite chambre d'aspiration (27).

8. Un dispositif selon la revendication 2, dans lequel lesdits moyens de filtration (29) comprennent une pluralité de premier micro-canaux longitudinaux (42) formés en positions décalées angulairement sur la paroi interne (43) de ladite partie d'extrémité distale (34) dudit trou (20), et au moins un premier joint d'étanchéité annulaire (39) reçu dans un premier logement annulaire (40) de la partie extrémité distale (36) de ladite tige (21) et apte à entrer en interaction avec lesdits premiers micro-canaux (42) pour former un premier filtre à labyrinthe (44).

9. Un dispositif selon la revendication 8, dans lequel lesdits moyens de filtration (29) comprennent une pluralité de deuxièmes micro-canaux longitudinaux (45) formés en positions décalées angulairement sur la paroi interne (43) de ladite partie proximale (35) dudit trou (20), et au moins un deuxième joint d'étanchéité annulaire (46) reçu dans un deuxième logement annulaire (47) de la partie proximale (48) de ladite tige (21) et apte à entrer en interaction avec lesdits deuxièmes micro-canaux (45) pour former un deuxième filtre à labyrinthe (49), décalé longitudinalement par rapport au premier.

10. Un dispositif selon la revendication 2, dans lequel ladite tige (21) possède un élément de verrouillage (53) situé près de ladite extrémité proximale (48) pour solidariser de façon amovible ledit piston (11) à ladite tige (21), celle-ci étant dans la position avant, et pour permettre audit piston (11) d'effectuer un mouvement de va-et-vient pour créer une pression négative dans la dite chambre d'aspiration (27) et pour alimenter de l'air dans ledit espace d'air (32), ledit élément de verrouillage (53) pouvant être relâché pour déplacer ladite tige (21) dans ladite position arrière, pour permettre la sortie de l'air collecté dans ledit espace d'air (32) à travers une ouverture d'aération (52).

11. Une procédé de perfusion de biomatériaux poreux (P) avec des liquides biologiques (L), comprenant les étapes suivantes :
a) réaliser un corps tubulaire (2) avec une cavité axiale (3) définissant une chambre de perfusion (4) ;
b) positionner un biomatériau poreux (P) dans ladite chambre (4) ;
c) remplir ladite chambre (4) avec un liquide de perfusion (L) ;
d) introduire un piston à mouvement axial (11), comportant un trou débouchant axial (20) dans ladite cavité (3) ;
e) loger une tige (21) dans ledit trou (20) avec un jeu prédéterminé pour définir un espace de collecte d'air (32) en communication avec ladite chambre (4) et avec l'extérieur et comportant des moyens de commande (33) pour commander la communication avec l'extérieur ;
f) positionner ladite tige (21), par rapport audit piston (11), dans une position avant de verrouillage afin d'empêcher le passage d'air depuis l'espace d'air (32) à l'extérieur ;
g) effectuer un mouvement de va-et-vient de l'ensemble tige-piston en condition de verrouillage pour créer des aspirations et des compressions répétées dans ladite chambre (4) et déterminer la séparation des bulles d'air du liquide et le transport de celles-ci vers ledit espace de collecte d'air (32) ;
h) positionner ladite tige (21) par rapport audit piston (11) dans une position arrière de déverrouillage pour permettre la communication dudit espace d'air (32) avec l'extérieur et libérer l'air collecté dans celui-ci ;
i) répéter les étapes g) et h) pour un nombre de fois nécessaire à compléter approximativement l'élimination d'air des pores du biomatériau poreux (P) et remplir ceux-ci avec le liquide de perfusion (L) ;
j) évacuer le liquide (L) de la chambre de perfusion (4) ;
k) ouvrir la chambre de perfusion (4) et enlever le matériau poreux complètement traité (P).

12. Une procédé selon la revendication 11, dans lequel une pluralité de micro-canaux longitudinaux (42, 45) sont agencés à l'entrée et à la sortie dudit espace d'air (32), et sont formés dans les zones d'extrémité (34, 35) dudit trou axial (20), et un ou plusieurs joints d'étanchéité annulaires (39, 49) sont agencés sur les zones correspondantes (36, 48) de ladite tige (21) pour former des ouvertures micrométriques, des étapes de filtration l) étant effectuées, dans lesquelles le liquide de perfusion (L) est filtré par lesdites ouvertures micrométriques pour empêcher la fuite des liquides et permettre le passage sélectif d'air.

13. Une procédé selon la revendication 11, dans lequel ladite tige (21) est verrouillée par rapport audit piston (11) au moyen d'un élément de verrouillage séparable (53) qui est situé près de l'extrémité proximale (16) du piston (11) et est conçu pour être accessible à un utilisateur.

14. Une procédé selon la revendication 11, dans lequel le mouvement de va-et-vient de ladite tige (21) dans le trou axial (20) est obtenu par la réalisation d'une bague d'actionnement (50) reliée à l'extrémité proximale (48) de ladite tige (21) et le mouvement longitudinal de celle-ci par l'utilisateur.

15. Une procédé selon la revendication 14, dans lequel un passage annulaire (23) est formé entre ledit espace d'air (32) et l'extérieur au niveau de ladite extrémité proximale (16) dudit piston (11) et un logement (22) pour ladite bague (50) est réalisé à la sortie dudit passage (23),
ledit passage (23) étant fermé par le mouvement vers l'avant ladite tige (21) et l'introduction de ladite bague (50) entière dans ledit logement (22), l'air collecté dans ledit espace d'air (32) étant libéré à l'extérieur par le mouvement vers l'arrière de ladite tige (21) et l'éloignement de ladite bague (50) dudit logement (22), ce qui comporte l'ouverture dudit passage (23).
